# EUROPEAN PATENT APPLICATION

(11) **EP 4 574 127 A1**
(43) Date of publication of application: **25.06.2025**
(21) Application number: 23855166.7
(22) Date of filing: 17.08.2023
(51) Int. Cl.: A61H 1/02

(54) **HIP JOINT AND ANKLE LINKING-TYPE WALKING ASSISTANCE APPARATUS**

(30) Priority: 19.08.2022 KR 20220103791
(71) Applicant: Seoul National University R & DB Foundation, Seoul 08826 (KR)
(72) Inventor: CHO, Kyu Jin, Seoul 06278 (KR); JUNG, Bong Keun, Seoul 08826 (KR); YUN, Sung Sik, Seoul 08801 (KR); YU, Ki Pyeong, Seoul 04309 (KR)
(74) Representative: EP&C
(86) International application number: PCT/KR2023/012202
(87) International publication number: WO 2024/039200

(57) **Abstract**

The present invention relates to a hip joint and ankle linking-type walking assistance apparatus, and more specifically, to a hip joint and ankle linking-type walking assistance apparatus which is worn on a user's body and assists the walking motion of the user according to the user's movement. The hip joint and ankle linking-type walking assistance apparatus of the present invention effectively links the movement of the hip joints and the movement of the ankle joints according to the structure of the human body, and thus has the effect of naturally assisting the walking motion while being small and light and convenient to wear.

## Description

### Technical Field

The present disclosure relates to a hip joint and ankle connecting type gait assistance apparatus, in more detail, a hip joint and ankle connecting type gait assistance apparatus that is worn on the body of a user and assists a gait motion following movement of the user.

### Background Art

Patients, such as stroke patients, whose ankle joints stiffen or contract due to involuntary motions of muscles have difficulty in actively moving their ankle joints in a gait process in many cases. Accordingly, such patients do not put their ankles down and push the ground or lift their ankles well in a gait process, so their gait motion is unnatural and unstable. The symptom that an ankle is not lifted and always dangles down is called foot drop. Patients with such a foot drop symptom frequently drag their feet on the floor or trip on obstacles. This not only causes injuries to the ankles and the parts around the ankles of a patient, but may be a factor that interferes with rehabilitation training.

Splint type devices that fix an ankle at a neutral position to prevent these problems are used to assist a gait in some cases. However, since such devices fix an ankle rather than help an ankle move in a normal pattern, the devices do not make harmony of several joints, which is required for a gait, and still make unnatural postures. That is, devices such as a splint that fixes an ankle are not suitable for assisting a gait of a patient or performing rehabilitation training and are usually used to prevent injuries or deformation of joints.

Robot type ankle assistance devices that are worn on the body of a user have been developed, but there is a problem that such active robot type devices are difficult to wear and inconvenient to use. Further, such devices according to the related art have a problem that the devices are not suitable for use in the daily life and the manufacturing cost is also high because they are big and heavy.

### Disclosure

### Technical Problem

The present disclosure has been made in an effort to satisfy the necessities described above and an objective of the present disclosure is to provide a hip joint and ankle connecting type gait assistance apparatus that effectively assists a gait and is convenient to use by being worn on the body of the user and helping movement of the ankle of the user.

### Technical Solution

In order to achieve the objectives described above, a hip joint and ankle connecting type gait assistance apparatus of the present disclosure is characterized by including: a waist strap configured to be worn around a waist; a mid-strap configured to be worn around a knee; a first sheath member installed on the mid-strap on a side of a knee; a fore foot strap configured to be worn on a front part of a foot; and a first wire fixed at a first end to the waist trap at a rear part of a waist and fixed at a second end to the fore foot strap through the first sheath member such that the first wire can slide while a path thereof is guided by the first sheath member.

### Advantageous Effects

The hip joint and ankle connecting type gait assistance apparatus effectively links movement of a hip joint and movement of an ankle joint in accordance with the structure of a human body, so there is an effect that the apparatus is small, light, and convenient to wear and can naturally assist a gait motion.

### Description of Drawings

FIGS. 1 to 3 are side views showing the state in which a user wears a hip joint and ankle connecting type gait assistance apparatus according to an embodiment of the present disclosure; and
FIG. 4 is an enlarged view showing a portion of the hip joint and ankle connecting type gait assistance apparatus shown in FIGS. 1 to 3.

### Best Mode

Hereafter, a hip joint and ankle connecting type gait assistance apparatus according to an embodiment of the present disclosure is described with reference to the accompanying drawings.

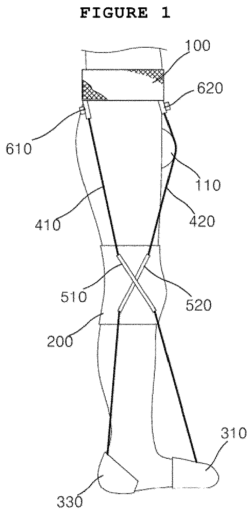

FIGS. 1 to 3 are side views showing the state in which a user wears a hip joint and ankle connecting type gait assistance apparatus according to an embodiment of the present disclosure.

Referring to FIG. 1 to 3, a hip joint and ankle connecting type gait assistance apparatus of this embodiment includes a waist strap 100, a mid-strap 200, a fore foot strap 310, a rear foot strap 330, a first wire 410, and a second wire 420.

The waist strap 100 is made of leather or fabric. The waist strap 100 is formed like a waist belt to be able to worn and fixed on the waist of a user. The first wire 410 and the second wire 420 are connected to the waist strap 100, so tension is transmitted to the waist strap 100. Accordingly, the waist strap 100 is formed in a structure that can be fixed on the waist of a user relatively firmly enough to be able to resist the tension that is transmitted from the first wire 410 or the second wire 420.

The mid-strap 200 is worn around a knee. The mid-strap 200 is formed in a similar shape to a knee protector and is worn around a knee of a user. The mid-strap 200 may be formed in a structure and a shape that do not interfere with a user bending and straightening a knee.

The fore foot strap is worn on the front part of a user's foot. The fore foot strap 310 may be directly worn and fixed on a foot of a user or may be installed in a shoe and indirectly worn.

The rear foot strap 330 is formed in a similar structure to the fore foot strap 310 and is worn on the rear part of a user's foot. The fore foot strap 310 may be worn at any position of a front part from an ankle joint of a user and is better to be positioned at the toes of a foot if possible. The rear foot strap 330 may be worn at any position of a rear part from an ankle joint of a user and it is better to position the rear foot strap 330 at the heel of a foot if possible.

The first wire 410 is installed to connect the waist strap 100 and the fore foot strap 310 to each other. A first end of the first wire 410 is fixed to the waist strap 100 and a second end thereof is fixed to the fore foot strap 310. The first wire 410 is better to be fixed to the waist strap 100 at the rear part of a waist to be able to go across the mid-strap 200 and a side of a knee.

The second wire 420 is installed to connect the waist strap 100 and the rear foot strap 330 to each other. A first end of the second wire 420 is fixed to the waist strap 100 and a second end thereof is fixed to the rear foot strap 330. The second wire 420 is better to be fixed to the waist strap 100 at the front part of a waist to be able to go across the mid-strap 200 and a side of a knee.

A first sheath member 510 and a second sheath member 520 are installed on a side of the mid-strap 200. In this case, the first sheath member 510 and the second sheath member 520 are each formed in a cylindrical shape like a straw. The first wire 410 and the second wire 420 are disposed slidably (relatively movably) through the first sheath member 510 and the second sheath member 520, respectively. The first sheath member 510 and the second sheath member 520 are disposed in the extension directions of the first wire 410 and the second wire 420, respectively, and fixed to the mid-strap 200 to be able to guide sliding of the first wire 410 and the second wire 420 while preventing the first wire 410 and the second wire 420 from separating from the mid-strap 200. Accordingly, the first sheath member 510 is disposed at an angle to extend forward as it goes down and the second sheath member 520 is disposed at an angle to extend rearward as it goes down.

FIGS. 1 to 3 show the state in which the first wire 410 and the second wire 420 are installed to go across the outside of a right leg.

In this embodiment, the first wire 410 is fixed to the waist strap 100 through a first adjustment member 610 and the second wire 420 is fixed to the waist strap 100 through a second adjustment member 620. In this embodiment, the first adjustment member 610 and the second adjustment member 620 are configured to adjust and fix the lengths of the first wire 410 and the second wire 420.

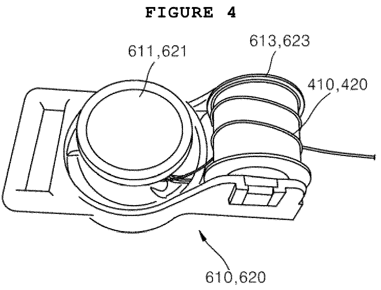

Referring to FIG. 4, the first adjustment member 610 includes a first BOA dial 611 and a first one-way clutch 613. The first BOA dial 611 is formed like a pulley and is configured to be able to wind or unwind the first wire 410. A user fixes the length of the first wire 410 by pressing the first BOA dial 611 after appropriately adjust the length of the first wire 410 by rotating the first BOA dial 611. The first wire 410 is partially wound around the first one-way clutch 513 and then connected to the first BOA dial 611. The first one-way clutch 613 serves to prevent damage to the first BOA dial 611 by resisting most of the tension applied to the first wire 410. The first one-way clutch 613 is formed in a structure that is freely rotated in the winding direction of the first wire 410 around the first BOA dial 611 and is not rotated in the opposite direction. Accordingly, when tension is applied to the first wire 410, the one-way clutch 613 resists the tension without rotating. When the first BOA dial 611 is rotated to reduce the length of the first wire 410, the first one-way clutch 613 is rotated, thereby allowing the first wire 410 to be wound around the first BOA dial 611.

The second adjustment member 620 is composed of a second BOA dial 621 and a second one-way clutch 623. The configuration and operation of the second adjustment member 620 are the same as those of the first adjustment member 610.

A protruding member 110 is installed at the waist strap 100. The protruding member 110 is disposed to be positioned on a thigh of a user and protrudes with respect to the thigh. The protruding member 110 is fixed to a separate fabric strap extending from the waist strap 100, whereby it is disposed at the position described above. The second wire 420 extends from the waist strap 110 to the rear foot strap 330 through the protruding member 110 and the second sheath member 520.

Hereafter, the operation of the hip joint and ankle connecting type gait assistance apparatus having the configuration described above in accordance with this embodiment is described.

FIG. 1 shows the state in which a user stands upright in a stop state. The lengths of the first wire 410 and the second wire are better to be set such that tension is not applied to the wires when a user stands upright, as described above, to this end, the lengths of the first wire 410 and the second wire 420 are adjusted by operating the first adjustment member 610 and the second adjustment member 620, respectively. Depending on cases, it may be possible to adjust the lengths of the first wire 410 and the second wire 420 into lengths that provide a little more room, as compared with when a user stands upright.

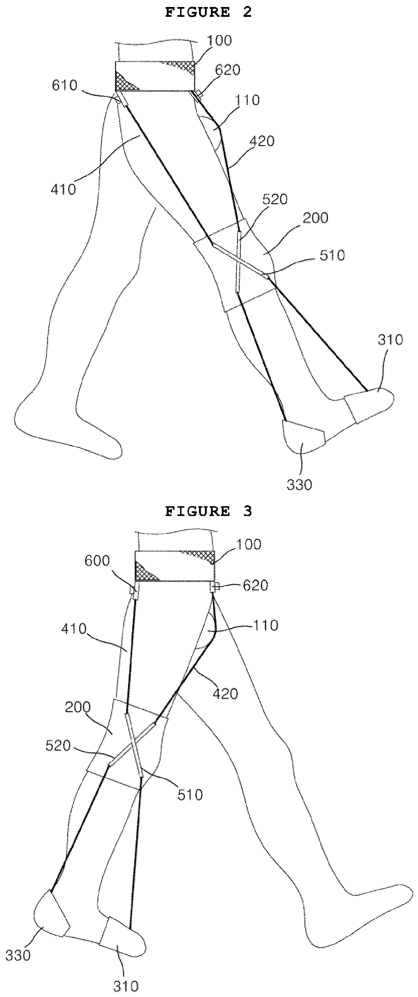

When the user starts to move and stretches the right foot forward in this state, it becomes the state shown in FIG. 2. The first wire 410 is operated when the right hip joint is bent while the right foot is stretched forward. When the right hip joint is bent, the right foot is stretched forward and the distance between the rear part of the waist and the end of the right foot increases. Since the length of the first wire 410 is limited, the first wire 410 pulls the fore foot strap 310 when the distance between the rear part of the waist and the end of the right foot starts to increase. As a result, the right ankle of the user is bent forward by the first wire 410, thereby inducting a natural gait of the user. When a use has a disorder or an injury such as foot drop at the right ankle and does not wear the hip joint and ankle connecting type gait assistance apparatus according to the present disclosure, the ankle joint of the user is not appropriately bent, so an injury may be generated. In particular, when foot drop is generated due to lack of power of an ankle, a toe trips on the floor and the danger of an injury increases when the user walks. However, since the first wire 410 pulls the fore foot strap 310 while a user bends a hip joint and stretches a foot forward, as described above, the ankle joint of the user is naturally induced to be bent, so the user's foot touches the ground from the heel, whereby it is possible to achieve an effect of assisting a gait of the user and preventing an injury.

The present disclosure has an important characteristic in that the first wire 410 is not fixed to the mid-strap 200 and is configured to move relatively to the mid-strap 200 through the first sheath member 510. Since the first wire 410 moves while sliding through the first sheath member 510, the first wire 410 can naturally operate in synchronization with movement of a hip joint and an ankle of a user. That is, even though the distance between the rear part of a waist and a knee joint and the distance between a knee joint and a toe are changed by movement of a user, the mid-strap 200 induce an ankle of the user to be bent along with movement of the user without limiting the length or movement of the first wire 410. The first sheath member 510 keeps the first wire 410 stuck to a side of a knee of a user and allows the first wire 410 to perform the function of gait assistance and gait induction. In particular, as described above and shown in the figures, when the first sheath member 510 is fixed to the mid-strap 200 at an angle in the extension direction of the first wire 410, this operation of the first wire 410 can be effectively induced.

Since the distance between the front part of a wait and the right heel decreases when a user stretches the right foot forward, as described above, tension is not applied to the second wire 420. Accordingly, when a hip joint is bent, the second wire 420 does not interfere with movement of a user.

Next, when a user keeps walking and stretches the left foot forward and the right foot backward, it becomes the state shown in FIG. 3.

This state is opposite to the state described with reference to FIG. 2. As the right hip joint is stretched, the distance between the front part of the waist and the right heel increases and the distance between the rear part of the waist and the right toe decreases. Accordingly, tension is not applied to the first wire 410 but is applied to the second wire 420. As a result, the second wire 420 induces a stretch of the right ankle while pulling the rear foot strap 330. The user obtains energy that enables the user to move forward while pushing the ground with the toe of the right foot that is the rear foot by the tension of the second wire 420. Accordingly, when the power of an ankle of a user is weak due to a disorder or an injury at the ankle, the second wire 420 assists the ligaments or muscles around the ankle joint, so the user can smoothly walk or run. Even in this case, similar to the case of the first wire 410, the second wire 420 is relatively moved while sliding through the second sheath member 520, so the second wire 420 operates effectively in synchronization with movement of the lower body of the user while not applying unnecessary force to the knee and the knee joint of the user.

As described above, the hip joint and ankle connecting type gait assistance apparatus of the present disclosure assists a gait motion of a user kinematically using the phenomenon that the distance between the rear part of a waist and a toe and the distance between the front part of a waist and a heel are changed by movement of a hip joint of a user. The present disclosure has an advantage that the apparatus can very effectively assist a gait of a user by acting like a passive actuator even without using a specific active actuator. According to the present disclosure, it is possible to achieve a small-size and light apparatus having a simple structure by having such a passive structure, so it is possible to minimize inconvenience due to wearing of a user and achieve a very excellent assistance effect in comparison to active assistance devices of the related art. Accordingly, a user can be greatly helped while preventing an injury by using the present disclosure. Further, it is possible to receive help in effectively using energy following a gait even through the user does not have an injury or a disorder.

Meanwhile, as shown in FIG. 3, when the protruding member 110 is disposed on the front of a thigh and the second wire 420 is installed to extend downward through the protruding member 110, it is possible to receive much help. Tension applied to the second wire 420 by the protruding member 110 may increase the length of a moment arm that is applied to the rear foot strap 330, a user moves forward while pushing the ground with a larger force by receiving help from the second wire 420 and the protruding member 110. As a result, a user can use a larger force as propelling power when walking or running.

A preferred embodiment of the present disclosure was described above, but the scope of the present disclosure is not limited to the above description and that shown in the figures.

For example, it was described above that the first wire 410 and the second wire 420 are fixed to the waist strap 100 through the first adjustment member 610 and a second adjustment member 620 each having a BOA dial structure, respectively, but they may be connected to the waist strap through components having other structures. It is also possible to configure a hip joint and ankle connecting type gait assistance apparatus having a structure in which the first wire and the second wire are directly connected to the waist strap without the first adjustment member 610 and a second adjustment member 620. Further, it is also possible to connect the first wire and the second wire to the waist strap using the second adjustment member 620 and a length adjustment device having a different structure from the second adjustment member 620.

Further, the first wire 410 and the second wire 420 may be made of an elastic material or may be formed to have elasticity at only a portion thereof. When first wire 410 and the second wire 420 have appropriate elasticity in this way, the force that assists an ankle joint of a user may somewhat decrease, but an effect that the wires operate more naturally in synchronization with movement of a user can be obtained. Depending on cases, the first wire and the second wire may be made of a less elastic steel wire and may be connected to the waist strap using a first adjustment member and a second adjustment member, which are operated in a spiral spring type, respectively. In this case, the first adjustment member and the second adjustment member transmit tension following length variation of the first wire and the second wire while elastically winding the first wire and the second wire, respectively.

Further, a hip joint and ankle connecting type gait assistance apparatus having a structure in which, as shown in FIGS. 1 to 3, the first wire 410, the second wire 420, the first sheath member 510, the second sheath member 520, etc. are disposed on the outside of a right leg was exemplified in the above description, but the disposition structure of the first wire 410, the second wire 420, the first sheath member 510, and the second sheath member 520 may be changed in various ways, if necessary. A separate set of a first wire, a second wire, a first sheath member, and a second sheath member may be disposed on the inside in addition to the outside of a leg, and a first wire, a second wire, a first sheath member, a second sheath member, etc. may be disposed on both legs.

Further, the structures and shapes of the waist strap, the mid-strap, the fore foot strap, the rear foot strap, etc. may be changed in various ways, depending on the body shape, preferred wearing type, etc. of users.

Further, a hip joint and ankle connecting type gait assistance apparatus having the protrusion member 110 was exemplified in the above description, it is also possible to configure a hip joint and ankle connecting type gait assistance apparatus without the protruding member 110.

Further, a hip joint and ankle connecting type gait assistance apparatus having a structure including the first wire 410, the second wire 420, the first sheath member 510, the second sheath member 520, etc. was exemplified in the above description, but it is also possible to configure a hip joint and ankle connecting type gait assistance apparatus not including the first wire 410, the first sheath member 510, and the fore foot strap 310 or not including the second wire 420, the second sheath member 520, and the rear foot strap 330. When only one of preventing leg drop of a user and assisting the force that pushes the ground by a user is required, it is possible to configure a hip joint and ankle connecting type gait assistance apparatus having a structure not including some components as described above.

### Industrial Applicability

The present invention effectively links the movement of the hip joints and the movement of the ankle joints according to the structure of the human body, and thus has the effect of naturally assisting the walking motion while being small and light and convenient to wear.

## Claims

1. A joint and ankle connecting type gait assistance apparatus comprising:
a waist strap configured to be worn around a waist;
a mid-strap configured to be worn around a knee;
a first sheath member installed on the mid-strap on a side of a knee;
a fore foot strap configured to be worn on a front part of a foot; and
a first wire fixed at a first end to the waist trap at a rear part of a waist and fixed at a second end to the fore foot strap through the first sheath member such that the first wire can slide while a path thereof is guided by the first sheath member.

2. The joint and ankle connecting type gait assistance apparatus of claim 1, further comprising:
a second sheath member installed on the mid-strap on a side of a knee;
a rear foot strap configured to be worn on a rear part of a foot; and
a second wire fixed at a first end to the waist trap at a front part of a waist and fixed at a second end to the rear foot strap through the second sheath member such that the second wire can slide while a path thereof is guided by the second sheath member.

3. The joint and ankle connecting type gait assistance apparatus of claim 2, wherein the first sheath member and the second sheath member are each formed in a cylindrical shape,
the first sheath member is disposed at an angle to extend forward as it goes downward, and
the second sheath member is disposed at an angle to extend rearward as it goes downward.

4. The joint and ankle connecting type gait assistance apparatus of claim 2, further comprising:
a first adjustment member connected between the first wire and the waist strap and configured to adjust a length of the first wire; and
a second adjustment member connected between the second wire and the waist strap and configured to adjust a length of the second wire.

5. The joint and ankle connecting type gait assistance apparatus of claim 4, wherein the first adjustment member includes a first BOA dial to which the first wire is connected and around which the first wire is wound, and a first one-way clutch formed such that the first wire is wound, and configured such that rotation is allowed only in a direction in which the first wire is wound toward the first BOA dial, and
the second adjustment member includes a second BOA dial to which the second wire is connected and around which the second wire is wound, and a second one-way clutch formed such that the second wire is wound, and configured such that rotation is allowed only in a direction in which the second wire is wound toward the second BOA dial.

6. The joint and ankle connecting type gait assistance apparatus of claim 2, further comprising a protruding member disposed to protrude with respect to a thigh between the waist strap and the mid-strap and configured to support the first wire to be able to increase a moment arm of tension of the second wire.

7. The joint and ankle connecting type gait assistance apparatus of claim 2, wherein the first wire and the second wire are at least partially made of an elastic material.

8. The joint and ankle connecting type gait assistance apparatus of claim 2, further comprising:
a first adjustment member connected between the first wire and the waist strap and configured to elastically wind the first wire in a spiral spring type; and
a second adjustment member connected between the second wire and the waist strap and configured to elastically wind the second wire in a spiral spring type.

9. The joint and ankle connecting type gait assistance apparatus of claim 8, wherein the first adjustment member includes a first BOA dial to which the first wire is connected and around which the first wire is wound, and a first one-way clutch formed such that the first wire is wound, and configured such that rotation is allowed only in a direction in which the first wire is wound toward the first BOA dial, and
the second adjustment member includes a second BOA dial to which the second wire is connected and around which the second wire is wound, and a second one-way clutch formed such that the second wire is wound, and configured such that rotation is allowed only in a direction in which the second wire is wound toward the second BOA dial.
